# EUROPEAN PATENT SPECIFICATION

(11) **EP 2 735 338 B1**
(45) Date of publication and mention of the grant of the patent: **06.02.2019**
(21) Application number: 14000597.6
(22) Date of filing: 04.09.2009
(51) Int. Cl.: A61P 25/04, A61K 31/137

(54) **PHARMACEUTICAL COMBINATION OF 3-(3-DIMETHYLAMINO-1-ETHYL-2-METHYL-PROPYL)-PHENOL AND PREGABALIN OR GABAPENTIN**
PHARMAZEUTISCHE KOMBINATION VON 3-(3-DIMETHYLAMINO-1-ETHYL-2-METHYL-PROPYL)-PHENOL MIT PREGABALIN ODER GABAPENTIN
COMBINAISON PHARMACEUTIQUE DE 3-(3-DIMETHYLAMINO-1-ETHYL-2-METHYL-PROPYL)-PHENOL AVEC PREGABALIN OU GABAPENTIN

(30) Priority: 05.09.2008 EP 08015625
(43) Date of publication of application: 28.05.2014
(62) Divisional of application: 09778335.1
(73) Proprietor: Grünenthal GmbH, 52078 Aachen (DE)
(72) Inventor: Schiene, Klaus, D-41363 Jüchen (DE); Bloms-Funke, Petra, D-52146 Würselen (DE); Christoph, Thomas, D-52080 Aachen (DE)
(74) Representative: Brosch, Oliver

(56) References cited:
- WO-A2-01/13904
- WO-A2-2007/005716
- WO-A2-2007/087452
- WO-A2-2008/027442
- WO-A2-2009/067703

## Description

The present invention relates to a combination comprising as components (a) at least one 3-(3-Dimethylamino-1-ethyl-2-methyl-propyl)-phenol compound, and (b) at least one antiepileptic, a pharmaceutical formulation and a dosage form comprising said combination as well as a method of treating pain, e.g. neuropathic pain, wherein components (a) and (b) are administered simultaneously or sequentially to a mammal, whereby component (a) may be administered before or after component (b) and whereby components (a) or (b) are administered to the mammal either via the same or a different pathway of administration.

The treatment of chronic and acute pain conditions is extremely important in medicine. There is currently a worldwide demand for additional, not exclusively opioid-based, but highly effective pain treatment. The urgent need for action for patient-oriented and purposeful treatment of pain conditions, this being taken to mean the successful and satisfactory treatment of pain for the patient, is documented in the large number of scientific papers which have recently appeared in the field of applied analgesics and fundamental research work on nociception.

Even if the analgesics that are currently used for treating pain, for example opioids, NA- and 5HT-reuptake inhibitors, NSAIDS and COX inhibitors, are analgesically effective, side effects nevertheless sometimes occur. WO 01/13904 describes substance combinations comprising a tramadol material and an anticonvulsant drug, which show super-additive effects upon administration. Due to the super-additive effect the overall dose and accordingly the risk of undesired side effects can be reduced.

Thus, it was an object of the present invention to find further combinations having improved properties. It was also an object of the present invention to find further combinations that are suitable for the treatment of pain and which preferably exhibit fewer undesired side effects compared to its individual components, if administered in effective doses.

It has been found that a combination comprising (a) at least one 3-(3-Dimethylamino-1-ethyl-2-methyl-propyl)-phenol compound, and (b) at least one antiepileptic exhibits an analgesic effect. If these components are present in the composition in such a weight ratio that a supra-additive or synergistic effect is observed upon administration to the patients, the overall administered dose may be lowered, so that fewer undesired side-effects will occur.

Accordingly, the present invention relates to a pharmaceutical combination comprising as components
(a) the compound (1R,2R)-3-(3-Dimethylamino-1-ethyl-2-methyl-propyl)-phenol of formula (I'), 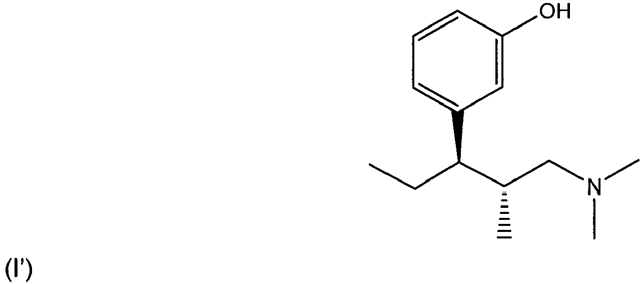 or an acid addition salt thereof, and
(b) at least one antiepileptic selected from the group consisting of pregabalin and gabapentin,
with the proviso that (1R,2R)-3-(3-Dimethylamino-1-ethyl-2-methyl-propyl)-phenol of formula (I') is not a slow release tapentadol.

The compound 3-(3-Dimethylamino-1-ethyl-2-methyl-propyl)-phenol of formula (I), its stereoisomers and corresponding salts thereof as well as methods for their preparation are well known, for example, from US 6,248,737 B1.

The compound 3-(3-Dimethylamino-1-ethyl-2-methyl-propyl)-phenol according to component (a) may be present in the inventive pharmaceutical composition in form of an acid addition salt, whereby any suitable acid capable of forming such an addition salt may be used.

The conversion of 3-(3-Dimethylamino-1-ethyl-2-methyl-propyl)-phenol into a corresponding addition salt, for example, via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example, diethyl ether, diisopropyl ether, alkyl acetates, acetone and/or 2-butanone. Moreover, trimethylchlorosilane in aqueous solution is also suitable for the preparation of hydrochlorides.

Antiepileptics, which are often also referred to as anticonvulsants, are well known in the art and include, without limitation, barbiturates and derivatives, such as methylphenobarbital, phenobarbital, primidone, barbexaclone and metharbital; hydantoin derivatives such as ethotoin, phenytoin, amino(diphenylhydantoin) valeric acid, mephenytoin and fosphenytoin; oxazolidine derivatives such as paramethadione, trimethadione and ethadione; succinimide derivatives such as ethosuximide, phensuximide and mesuximide; benzodiazepine derivatives such as clonazepam; carboxamide derivatives such as carbamazepine, oxcarbazepine, eslicarbazepine and rufinamide; fatty acid derivatives such as valproic acid, valpromide, aminobutyric acid, vigabatrin, progabide and tiagabine; or other antiepileptics such as sultiame, phenacemide, lamotrigine, felbamate, topiramate, gabapentin, pheneturide, levetiracetam, brivaracetam, selectracetam, zonisamide, pregabalin, stiripentol, lacosamide and beclamide.

These afore mentioned classes of antiepileptics and most of their individual representatives are, for example, listed in the Anatomical Therapeutic Chemical (ATC) classification under [N03] as used by the WHO for classification of pharmaceutically active ingredients (preferred edition: January 2008 or 2009). With regard to further details of the ATC-index reference is made to U. Fricke, J. Günther, Anatomischtherapeutisch-chemische Klassifikation mit Tagesdosen für den deutschen Arzneimittelmarkt: Methodik der ATC-Klassifikation und DDD-Festlegung. ATC-Index mit DDD-Angaben, Wissenschaftliches Institut der AOK; and Swiss Pharmaceutical Society, Index Nominum: International Drug Directory, CRC Press; 18th edition (January 31, 2004).

Other suitable antiepileptics include, for example, mexiletin, retigabin and ralfinamide. Some antiepileptics are known to be useful in the treatment of neuropathic pain. In one embodiment of the present invention one or more of these antiepileptics is used as component (b).

Also included are stereoisomers, salts, solvates, polymorphs and derivatives of the antiepileptic component as well as mixtures of any of the foregoing.

The antiepileptic according to component (b) is selected from the group consisting of pregabalin, and gabapentin,

In another embodiment of the inventive combination the antiepileptic according to component (b) is pregabalin.

In yet another embodiment of the inventive combination the antiepileptic according to component (b) is (S)-pregabalin.

Another specific embodiment of the present invention is a combination comprising (a) (1R,2R)-3-(3-Dimethylamino-1-ethyl-2-methyl-propyl)-phenol, or an acid addition salt thereof such as the hydrochloride addition salt, and (b) pregabalin.

Yet another specific embodiment of the present invention is a combination comprising (a) (1R,2R)-3-(3-Dimethylamino-1-ethyl-2-methyl-propyl)-phenol, or an acid addition salt thereof such as the hydrochloride addition salt, and (b) (S)-pregabalin.

Some antiepileptics comprise functional groups, for example, acidic groups such as carboxy groups which are capable of forming salts with the 3-(3-Dimethylamino-1-ethyl-2-methyl-propyl)-phenol component of formula (I), thereby incorporating both components (a) and (b) in one and the same salt.

Thus, in another embodiment of the present invention the inventive combination comprises components (a) and (b) in form of a salt formed from these two components. Such a salt formation may be partial, i.e. the inventive composition comprises one or both of these components also in their non-salt form, or the salt formation may essentially be complete.

Both components (a) and (b) as part of the inventive combination may be administered in amounts up to their maximum daily dosage, which is known to those skilled in the art. The compound Pregabalin may preferably be administered to a patient in a daily dosage of 1 to 1200 mg, the compound Gabapentin may preferably be administered to a patient in a daily dosage of 1 to 5000 mg.

The compound (1R,2R)-3-(3-Dimethylamino-1-ethyl-2-methyl-propyl)-phenol may preferably be administered to a patient in a daily dosage of 25 to 1000 mg, particularly preferably in a dosage of 50 to 800 mg, more particularly preferably in a dosage of 100 to 600 mg. When administered as part of the inventive combination the administered amount per day of component (a) and/or component (b) may be less than the respective maximum daily dosage and be, for example, 75±15 wt.-%, 75±10 wt.-%, 75±5 wt.-%, 50±15 wt.-%, 50±10 wt.-%, 50±5 wt.-%, 25±15 wt.-%, 25±10 wt.-% and 25±5 wt.-% for each of the components.

In another embodiment of the present invention the inventive combination may contain components (a) and (b) essentially in an equieffective ratio.

In yet a further embodiment of the inventive combination components (a) and (b) are present in such a weight ratio that the resulting composition will exert a supra-additive or synergistic effect upon administration to a patient. Suitable weight ratios can be determined by methods well known to those skilled in the art.

Both components (a) and (b) may also be present in the inventive combination in ratios deviating from the equieffective ratio. For, example, each of the components could be present in a range from 1/50 of the equieffective amount to 50 times the equieffective amount, from 1/20 of the equieffective amount to 20 times the equieffective amount, from 1/10 of the equieffective amount to 10 times the equieffective amount, from 1/5 of the equieffective amount to 5 times the equieffective amount, from 1/4 of the equieffective amount to 4 times the equieffective amount, from 1/3 of the equieffective amount to 3 times the equieffective amount, or from 1/2 of the equieffective amount to 2 times the equieffective amount.

In another embodiment of the present invention the components (a) and (b) can be administered in a specific dosage regimen to treat pain, for example, neuropathic pain. Components (a) and (b) may be administered simultaneously or sequentially to one another, in each case via the same or different administration pathways.

Another aspect of the present invention is therefore a method of treating pain, characterized in that components (a) and (b) are administered simultaneously or sequentially to a mammal, wherein component (a) may be administered before or after component (b) and wherein components (a) or (b) are administered to the mammal either via the same or a different pathway of administration.

The term pain as used herein includes but is not limited to inflammatory pain, neuropathic pain, acute pain, chronic pain, visceral pain, migraine pain and cancer pain.

Suitable pathways of administration include but are not limited to oral, intravenous, intraarterial, intraperitoneal, intradermal, transdermal, intrathekal, intramuscular, intranasal, transmucosal, subcutaneous, and rectal administration.

The inventive combinations are toxicologically safe and are therefore suitable for the treatment of mammals, particularly humans including infants, children and grown-ups.

Thus, in a further aspect the present invention relates to a pharmaceutical composition comprising an inventive combination as described herein and one or more auxiliary agents.

In a further aspect the present invention relates to a pharmaceutical dosage form comprising an inventive combination as described herein and one or more auxiliary agents.

In one embodiment the inventive pharmaceutical dosage form additionally comprises caffeine.

In one embodiment, the inventive pharmaceutical dosage form is suitable for being administered orally, intravenously, intraarterially, intraperitoneally, intradermally, transdermally, intrathekally, intramuscularly, intranasally, transmucosally, subcutaneously, or rectally.

The inventive formulations and dosage forms may contain auxiliary agents, for example, carriers, fillers, solvents, diluents, colorants and/or binders. The selection of auxiliary agents and of the amounts of the same to be used depends, for example, on how the drug is to be administered, e.g. orally, intravenously, intraarterially, intraperitoneally, intradermally, transdermally, intramuscularly, intranasally or locally, for example for infections of the skin, of the mucous membranes or of the eye.

Suitable auxiliary agents in the context of this invention are in particular any substances known to a person skilled in the art useful for the preparation of galenical formulations. Examples of suitable auxiliary agents include but are not limited to: water, ethanol, 2-propanol, glycerol, ethylene glycol, propylene glycol, polyethylene glycol, polypropylene glycol, glucose, fructose, lactose, saccharose, dextrose, molasses, starch, modified starch, gelatine, sorbitol, inositol, mannitol, microcrystalline cellulose, methyl cellulose, carboxymethyl cellulose, cellulose acetate, shellac, cetyl alcohol, polyvinyl pyrrolidone, paraffins, waxes, natural and synthetic gums, acacia gum, alginates, dextran, saturated and unsaturated fatty acids, stearic acid, magnesium stearate, zinc stearate, glycerol stearate, sodium lauryl sulphate, edible oils, sesame oil, coconut oil, peanut oil, soybean oil, lecithin, sodium lactate, polyoxyethylene and polypropylene fatty acid ester, sorbitan fatty acid ester, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulphate, zinc sulphate, calcium sulphate, potash, calcium phosphate, dicalcium phosphate, potassium bromide, potassium iodide, talcum, kaolin, pectin, crosspovidone, agar and bentonite.

Pharmaceutical formulations (dosage forms) in the form of tablets, effervescent tablets, chewing tablets, dragees, capsules, drops, juices or syrups are, for example, suitable for oral administration. Oral pharmaceutical formulations may also be in the form of multiparticulates such as granules, pellets, spheres, crystals and the like, optionally compressed into a tablet, filled into a capsule, filled into a sachet or suspended in a suitable liquid medium. Oral pharmaceutical formulations may also be equipped with an enteric coating.

Pharmaceutical formulations that are suitable for parenteral, topical and inhalative administration include but are not limited to solutions, suspensions, easily reconstitutable dry preparations and sprays.

Suppositories are a suitable pharmaceutical formulation for rectal administration. Formulations in a deposit, in dissolved form, for example, in a patch optionally with the addition of agents to promote skin penetration, are examples of suitable formulations for percutaneous administration.

The inventive pharmaceutical formulations may be produced using materials, means, devices and processes that are well known in the prior art of pharmaceutical formulations, as described for example in "Remington's Pharmaceutical Sciences", A.R. Gennaro (ed.), 17th edition, Mack Publishing Company, Easton, Pa. (1985), in particular in part 8, chapters 76 to 93. In order to obtain a solid pharmaceutical formulation such as a tablet, for example, the components of the pharmaceutical composition may be granulated with a pharmaceutical carrier, for example conventional tablet ingredients such as corn starch, lactose, saccharose, sorbitol, talcum, magnesium stearate, dicalcium phosphate or pharmaceutically acceptable gums, and pharmaceutical diluents, for example water, in order to form a solid composition that contains the components in homogeneous distribution. The term "homogeneous distribution" is taken to mean that the components are distributed uniformly over the entire composition, so that said composition may easily be divided into equally effective unit dose forms, such as tablets, pills or capsules and the like. The solid composition is then divided into unit dose forms.

If component (a) is to be released prior to the other component, for example at least 30 minutes or 1 hour beforehand, pharmaceutical formulations having a corresponding release profile may be prepared. An example of such a formulation is an osmotically driven release system for achieving a delayed release of one component via a coating that itself contains the other component which is accordingly released earlier. In a release system of this kind, which is particularly suitable for oral administration, at least part, and preferably all, of the surface of the release system, preferably those parts that will come into contact with the release medium, is/are semipermeable, preferably equipped with a semipermeable coating, so the surface(s) is/are permeable to the release medium, but substantially, preferably entirely, impermeable to the active ingredient, the surface(s) and/or optionally the coating comprising at least one opening for releasing the active ingredient. Moreover, precisely that/those surface(s) that is/are in contact with the release medium is/are provided with a coating containing and releasing the other component. This is preferably taken to mean a system in tablet form comprising a release opening, an osmotic pharmaceutical composition core, a semipermeable membrane and a polymer portion that exerts pressure upon swelling. A suitable example of this kind of system is the system distributed by ALZA Corporation, USA under the tradenames OROS®, in particular, the OROS® Push-Pull™ System, the OROS® Delayed Push-Pull™ System, the OROS® Multi-Layer Push-Pull™ system, the OROS® Push-Stick System and also, in specific cases, the L-OROS™. Embodiments and examples of osmotically driven release systems are, for example, disclosed in US patents 4,765,989, 4,783,337 and 4,612,008,

A further example of a suitable pharmaceutical formulation is a gel-matrix tablet, such as the products developed by Penwest Pharmaceuticals (for example, under TimeRX). Suitable examples are provided in US patents 5,330,761, 5,399,362, 5,472,711 and 5,455,046, Particularly suitable is a retarding matrix formulation, with an inhomogeneous distribution of the pharmaceutically active composition, whereby, component (a) can be distributed in the outer region (the portion that comes into contact with the release medium most quickly) of the matrix and the other component is distributed inside the matrix. On contact with the release medium, the outer matrix layer initially (and rapidly) swells and firstly releases the first component, followed by the significantly (more) retarded release of the other component. Examples of a suitable matrix include matrices with 1 to 80 % by weight of one or more hydrophilic or hydrophobic polymers as pharmaceutically acceptable matrix formers. A further example of a suitable matrix may be inferred from US 4,389,393.

The amount of the inventive pharmaceutically active combination to be administered to the patient may vary depending on different factors well known to those skilled in the art, for example, the weight of the patient, the route of administration, or the severity of the illness.

In a further aspect the present invention relates to the use of an inventive combination as described herein for the treatment of pain, said pain preferably including but not being limited to inflammatory pain, neuropathic pain, acute pain, chronic pain, visceral pain, migraine pain and cancer pain.

In another aspect the present invention relates to the use of an inventive combination as described herein for the preparation of a medicament for the treatment of pain, said pain preferably including but not being limited to inflammatory pain, neuropathic pain, acute pain, chronic pain, visceral pain, migraine pain and cancer pain.

In another aspect the present invention relates to a method of treating pain in a mammal, preferably a human, which comprises administering an effective amount of an inventive combination as described herein to the mammal.

### Pharmacological methods:

### In vivo experiments according to Chung

The weight ratios of components (a) and (b) that will lead to a supra-additive effect/synergistic effect of the inventive compositions may be determined in the test of Kim & Chung as described in Kim SH, Chung JM. An experimental model for peripheral mononeuropathy produced by segmental spinal nerve ligation in the rat. Pain 1992; 50: 355-63. Said reference is hereby incorporated by reference and forms part of the disclosure.

Ligatures were applied to the left L5/L6 spinal nerves of male Sprague-Dawley rats (140-160 g body weight, Janvier, Genest St. Isle, France). Animals developed tactile allodynia at the ipsilateral paw. Three to four weeks after the operation the tactile allodynia threshold baseline (withdrawal threshold) was measured on the ipsilateral and contralateral hind paw by an electronic von Frey anaesthesiometer (Somedic, Schweden). After test and measurement of the baseline, (1R,2R)-3-(3-Dimethylamino-1-ethyl-2-methyl-propyl)-phenol hydrochloride (hereinafter referred to as tapentadol hydrochloride or tapentadol-HCI), (S)-pregabalin as well as the inventive combination of tapentadol-HCI and (S)-pregabalin were each dissolved in 0.9 % NaCl solution and injected by the intravenous (i.v.) route (application volume 5 ml/kg). Animals were randomly assigned to groups of 10 for each test dose and vehicle (0.9 % NaCl solution) and tactile withdrawal thresholds were tested 0.5 h before administration and on several time points (0.5, 1 and 3 hours) after intravenous administration. Ipsi- and contralateral hindpaws were tested. The median of the withdrawal threshold for each animal at a given time is calculated from five individual stimulations with the electronic von Frey filament. Withdrawal thresholds of the injured paws are expressed as % MPE (Maximum possible effect) comparing predrug threshold of Chung-Animals (= 0 % MPE) and control threshold of sham-animals (100 % MPE). A cut-off is set at 100 % MPE. The effect of each compound and vehicle is calculated for each testing time point as interindividual % MPE value.

Data (anti-allodynic efficacy (% MPE), ipsi-lateral, paw withdrawal threshold (g), ipsi- and conralateral) were analysed by means of a two-factor analysis of variance (ANOVA) with repeated measures. In case of a significant treatment effect, post hoc analysis with Bonferroni adjustment was performed. Results were considered statistically significant if P < 0.05. ED₅₀ values and 95 % confidence intervals (95 % CI) were determined for the anti-allodynic efficacy (% MPE) at the time of the peak effect for each drug by regression analysis.

The analysis of the results was carried out via statistical comparison of the theoretical additive ED₅₀-value with the experimentally determined ED₅₀-value of a so-called fixed ratio combination (isobolographic analysis according to Tallarida RJ, Porreca F, and Cowan A. Statistical analysis of drug-drug and site-site interactions with isobolograms. Life Sci 1989; 45: 947-61, which is hereby incorporated by reference and forms part of the present disclosure).

### Results:

Tapentadol hydrochloride (0.1, 0.316, 1, 3.16 and 10 mg/kg i.v.) showed a dose dependent increase in the withdrawal threshold of the ipsi-lateral hind paw with an efficacy of 94 % MPE and an ED₅₀-value (95 % confidence interval) of 1.65 (1.20 - 2.35) mg/kg i.v. calculated from the peak effect vs. control values at 30 min. after administration.

(S)-Pregabalin (0.1, 3.16 and 10 mg/kg i.v.) showed a dose dependent increase in the withdrawal threshold of the ipsi-lateral hind paw with an efficacy of 67% MPE and an ED₅₀-value (95 % confidence interval) of 4.20 (3.37 - 5.43) mg/kg i.v. calculated from the peak effect vs. control values at 30 min. after administration.

Tapentadol hydrochloride and (S)-pregabalin show a potency difference which amounts to a factor 2.5 based on the ED₅₀ values 30 minutes after administration. Combinations in a fixed ratio of 1 : 2.5 (tapentadol hydrochloride : (S)-pregabalin) were tested in doses of 0.1 mg/kg + 0.25 mg/kg; 0.3 mg/kg + 0.75 mg/kg, 1 mg/kg + 2.5 mg/kg i.v. tapentadol hydrochloride + (S)-pregabalin, respectively. These combinations showed a dose dependent increase in the withdrawal threshold of the ipsi-lateral hind paw. The highest dose combination tested showed full efficacy with 89 % MPE. Potency was quantified by an ED₅₀ value (95 % confidence interval) of 0.83 (0.74 - 0.92) mg/kg i.v. calculated from the peak effect vs control values at 30 min after administration.

The results of the isobolographic analysis are summarized in the following table 1.

**Table 1:**

| Experimental ED₅₀ values of tapentadol hydrochloride and (S)-pregabalin and isobolographic analysis of the interaction between tapentadol hydrochloride and (S)-pregabalin: | | | | | |
|---|---|---|---|---|---|
| | tapentadol-HCl | (S)-pregabalin | Theoretical ED₅₀ of combination of tapentadol-HCI and (S)-pregabalin | Experimental ED₅₀ of the combination of tapentadol-HCl and (S)-pregabalin | interaction |
| Substance / ED₅₀ [mg/kg i.v.] (95 % confidence interval) | 1.65 (1.20-2.35) | 4.20 (3.37 - 5.43) | 2.91 (2.28-3.54) | 0.83 (0.74-0.92) | synergistic (p<0.001) |

| | | | | | |
|---|---|---|---|---|---|
| p: Level of statistical significance | | | | | |

The experimental ED₅₀ value (95 % confidence interval) of 0.83 (0.74 - 0.92) mg/kg i.v. of the inventive combination is below the theoretical additive ED₅₀ value (95 % confidence interval) of 2.91 (2.28 - 3.54) mg/kg i.v. and is statistically significant (p<0.001) as compared to the line of additivity. Thus, the interaction of tapentadol hydrochloride and (S)-pregabalin is synergistic.

Analysis of contra-lateral paw withdrawal thresholds reveal significant anti-nociceptive effects of tapentadol hydrochloride and (S)-pregabalin at 10 mg/kg i.v. while no significant anti-nociceptive effect was seen at the highest dose of the inventive combination. Thus, synergistic anti-allodynic activity of tapentadol hydrochloride and (S)-pregabalin result in reduced anti-nociceptive side effects.

## Claims

1. A combination comprising as component(s):
(a) (1R,2R)-3-(3-Dimethylamino-1-ethyl-2-methyl-propyl)-phenol of formula (I'), 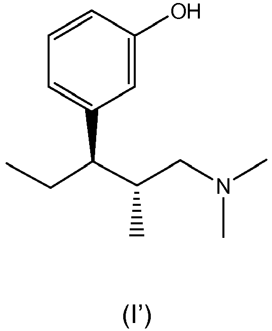 or an acid addition salt thereof, and
(b) at least one antiepileptic selected from the group consisting of pregabalin and gabapentin,
with the proviso that (1R,2R)-3-(3-Dimethylamino-1-ethyl-2-methyl-propyl)-phenol of formula (I') is not a slow release tapentadol.

2. The combination according to claim 1, whereby the acid addition salt is hydrochloride.

3. The combination according to claim 1 or 2, **characterized in that** components (a) and (b) are present as a salt formed from these two components.

4. The combination according to any of claims 1-3, **characterized in that** components (a) and (b) are present in such a weight ratio that the composition will exert a synergistic effect upon administration to a patient.

5. A pharmaceutical composition comprising a combination according to any one of claims 1-4 and optionally one or more auxiliary agents.

6. A dosage form comprising a combination according to any one of claims 1-4 and optionally one or more auxiliary agents.

7. The dosage form according to claim 6, **characterized in that** it is suitable for oral, intravenous, intraarterial, intraperitoneal, intradermal, transdermal, intrathekal, intramuscular, intranasal, transmucosal, subcutaneous, or rectal administration.

8. The dosage form according to claim 6 or 7, **characterized in that** one or both of the components (a) and (b) is/are present in controlled-release form.

9. The dosage form according to any one of claims 6-8, **characterized in that** it additionally comprises caffeine.

10. Use of a combination according to any one of claims 1-4 for the preparation of a medicament for the treatment of pain.

11. The use according to claim 10, **characterized in that** the pain is selected from inflammatory pain, neuropathic pain, acute pain, chronic pain, visceral pain, migraine pain and cancer pain.

12. The use according to claim 10 or 11, **characterized in that** the medicament is adapted for simultaneous or sequential administration of components (a) and (b), wherein compound (a) may be administered before or after compound (b) and wherein compounds (a) or (b) are administered to the mammal either by the same or a different pathway of administration.

13. Combination according to any of claims 1-4 for use in the treatment of pain.

## Patentansprüche

1. Kombination, umfassend als Komponente(n):
(a) (1R,2R)-3-(3-Dimethylamino-1-ethyl-2-methyl-propyl)-phenol der Formel (I'), 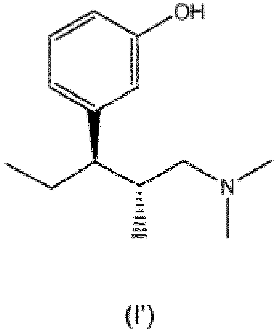 oder ein Säureadditionssalz davon, und
(b) mindestens ein Antiepileptikum, das ausgewählt ist aus der Gruppe, bestehend aus Pregabalin und Gabapentin,
mit der Maßgabe, dass es sich bei dem (1R, 2R)-3-(3-Dimethylamino-1-ethyl-2-methyl-propyl)-phenol der Formel (I') nicht um ein Tapentadol mit langsamer Freisetzung handelt.

2. Kombination nach Anspruch 1, wobei es sich bei dem Säureadditionssalz um Hydrochlorid handelt.

3. Kombination nach Anspruch 1 oder 2, **dadurch gekennzeichnet, dass** die Komponenten (a) und (b) als Salz vorliegen, das aus diesen beiden Komponenten gebildet ist.

4. Kombination nach einem der Ansprüche 1-3, **dadurch gekennzeichnet, dass** die Komponenten (a) und (b) in einem solchen Gewichtsverhältnis vorliegen, dass die Zusammensetzung bei Verabreichung an einen Patienten eine synergistische Wirkung ausübt.

5. Pharmazeutische Zusammensetzung, umfassend eine Kombination nach einem der Ansprüche 1-4 und gegebenenfalls einen oder mehrere Hilfsstoffe.

6. Darreichungsform, umfassend eine Kombination nach einem der Ansprüche 1-4 und gegebenenfalls einen oder mehrere Hilfsstoffe.

7. Darreichungsform nach Anspruch 6, **dadurch gekennzeichnet, dass** sie zur oralen, intravenösen, intraarteriellen, intraperitonealen, intradermalen, transdermalen, intrathekalen, intramuskulären, intranasalen, transmukosalen, subkutanen oder rektalen Verabreichung geeignet ist.

8. Darreichungsform nach Anspruch 6 oder 7, **dadurch gekennzeichnet, dass** eine oder beide der Komponenten (a) und (b) in einer Form mit kontrollierter Freisetzung vorliegt/vorliegen.

9. Darreichungsform nach einem der Ansprüche 6-8, **dadurch gekennzeichnet, dass** sie zusätzlich Koffein umfasst.

10. Verwendung einer Kombination nach einem der Ansprüche 1-4 zur Herstellung eines Medikaments zur Behandlung von Schmerz.

11. Verwendung nach Anspruch 10, **dadurch gekennzeichnet, dass** der Schmerz ausgewählt ist aus Entzündungsschmerz, neuropathischem Schmerz, akute, Schmerz, chronischem Schmerz, viszeralem Schmerz, Migräneschmerz und Krebsschmerz.

12. Verwendung nach Anspruch 10 oder 11, **dadurch gekennzeichnet, dass** das Medikament zur gleichzeitigen oder aufeinanderfolgenden Verabreichung der Komponenten (a) und (b) ausgelegt ist, wobei die Verbindung (a) vor oder nach der Verbindung (b) verabreicht werden kann und wobei die Verbindungen (a) oder (b) über den gleichen oder einen anderen Verabreichungsweg an den Säuger verabreicht werden.

13. Kombination nach einem der Ansprüche 1-4 zur Verwendung bei der Behandlung von Schmerz.

## Revendications

1. Combinaison comprenant en tant que constituant(s) :
(a) le (1R,2R)-3-(3-diméthylamino-1-éthyl-2-méthyl-propyl)-phénol de formule (I'), 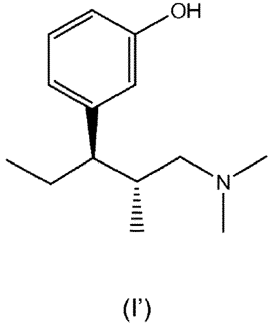 ou un sel d'addition d'acide de celui-ci, et
(b) au moins un antiépileptique sélectionné dans le groupe constitué de la prégabaline et de la gabapentine,
à condition que le (1R,2R)-3-(3-diméthylamino-1-éthyl-2-méthyl-propyl)-phénol de formule (I') ne soit pas un tapentadol à libération retardée.

2. Composition selon la revendication 1, dans laquelle le sel d'addition d'acide est le chlorhydrate.

3. Combinaison selon la revendication 1 ou 2, **caractérisée en ce que** les constituants (a) et (b) sont présents sous la forme d'un sel formé à partir de ces deux constituants.

4. Combinaison selon l'une quelconque des revendications 1 à 3, **caractérisée en ce que** les constituants (a) et (b) sont présents en un rapport en poids tel que la composition aura un effet synergique lors de son administration à un patient.

5. Composition pharmaceutique comprenant une combinaison selon l'une quelconque des revendications 1 à 4 et facultativement un ou plusieurs agents auxiliaires.

6. Forme galénique comprenant une combinaison selon l'une quelconque des revendications 1 à 4 et facultativement un ou plusieurs agents auxiliaires.

7. Forme galénique selon la revendication 6, **caractérisée en ce qu'**elle est appropriée pour une administration par voie orale, intraveineuse, intra-artérielle, intrapéritonéale, intradermique, transdermique, intrathécale, intramusculaire, intranasale, transmuqueuse, sous-cutanée ou rectale.

8. Forme galénique selon la revendication 6 ou 7, **caractérisée en ce que** l'un des constituants (a) et (b) ou les deux est/sont présent(s) sous une forme à libération contrôlée.

9. Forme galénique selon l'une quelconque des revendications 6 à 8, **caractérisée en ce qu'**elle comprend en outre de la caféine.

10. Utilisation d'une combinaison selon l'une quelconque des revendications 1 à 4 pour la préparation d'un médicament destiné au traitement de la douleur.

11. Utilisation selon la revendication 10, **caractérisée en ce que** la douleur est sélectionnée parmi une douleur inflammatoire, une douleur neuropathique, une douleur aiguë, une douleur chronique, une douleur viscérale, une douleur due à une migraine et une douleur due à un cancer.

12. Utilisation selon la revendication 10 ou 11, **caractérisée en ce que** le médicament est adapté à une administration simultanée ou séquentielle des constituants (a) et (b), dans laquelle le composé (a) peut être administré avant ou après le composé (b) et dans laquelle les composés (a) et (b) sont administrés au mammifère par une voie d'administration identique ou différente.

13. Combinaison selon l'une quelconque des revendications 1 à 4 pour son utilisation dans le traitement de la douleur.
